# EUROPEAN PATENT SPECIFICATION

(11) **EP 1 399 471 B1**
(45) Date of publication and mention of the grant of the patent: **30.01.2008**
(21) Application number: 02762309.9
(22) Date of filing: 27.06.2002
(51) Int. Cl.: C07K 7/08, A61K 38/04, A61K 38/08, A61P 25/16, A61P 25/28, A61P 25/06, A61P 25/00

(54) **USE OF DIPEPTIDYL PEPTIDASE IV INHIBITORS AS THERAPEUTICS FOR NEUROLOGICAL DISORDERS**
VERWENDUNG VON DPIV-INHIBITOREN ALS THERAPEUTIKA FÜR KRANKHAFTE NEUROLOGISCHE ZUSTÄNDE
UTILISATION D'INHIBITEURS DE LA DIPEPTIDYL PEPTIDASE IV COMME AGENTS POUR LA THERAPIE DES MALADIES NEUROLOGIQUES

(30) Priority: 27.06.2001 EP 01114796; 12.10.2001 DE 10150203; 09.11.2001 DE 10154689; 28.02.2002 US 360909 P
(43) Date of publication of application: 24.03.2004
(73) Proprietor: Probiodrug AG, 06120 Halle/Saale (DE)
(72) Inventor: DEMUTH, Hans-Ulrich, 06114 Halle/Saale (DE); HOFFMANN, Torsten, 06114 Halle/Saale (DE); HEISER, Ulrich, 06108 Halle/Saale (DE); GLUND, Konrad, 06120 Halle/Salle (DE); VON HÖRSTEN, Stephan, 30900 Wedemark (DE)
(74) Representative: Hoffmann, Matthias
(86) International application number: PCT/EP2002/007133
(87) International publication number: WO 2003/002596

(56) References cited:
- WO-A-01/34594
- WO-A-01/89569
- WO-A-99/62914
- REINHOLD DIRK ET AL: "Inhibitors of dipeptidyl peptidase IV/CD26 suppress activation of human MBP-specific CD4+ T cell clones." JOURNAL OF NEUROIMMUNOLOGY, vol. 87, no. 1-2, 1 July 1998 (1998-07-01), pages 203-209, XP002214116 ISSN: 0165-5728
- STOCKEL-MASCHEK^A A ET AL: "Thioxo amino acid pyrrolidides and thiazolidides: new inhibitors of proline specific peptidases" BIOCHIMICA ET BIOPHYSICA ACTA. PROTEIN STRUCTURE AND MOLECULAR ENZYMOLOGY, ELSEVIER, AMSTERDAM,, NL, vol. 1479, no. 1-2, 15 June 2000 (2000-06-15), pages 15-31, XP004279023 ISSN: 0167-4838

## Description

### Field Of The Invention

The present invention relates to inhibitors of dipeptidyl peptidase IV and dipeptidyl peptidase IV-like enzyme activity and, more particularly, pharmaceutical compositions containing said compounds, and the use of said compounds for the treatment of multiple sclerosis.

### Background Art

Dipeptidyl peptidase IV (DPIV) is a serine protease which cleaves N-terminal dipeptides from a peptide chain containing, preferably, a proline residue in the penultimate position. Although the biological role of DPIV in mammalian systems has not been completely established, it is believed to play an important role in neuropeptide metabolism, T-cell activation, and the entry of HIV into lymphoid cells.

Likewise, it has been discovered that DPIV is responsible for inactivating glucagon-like peptide-1 (GLP-1) and glucose-dependent insulinotropic peptide also known as gastric-inhibitory peptide (GIP). Since GLP-1 is a major stimulator of pancreatic insulin secretion and has direct beneficial effects on glucose disposal, in WO 97/40832 and US 6,303,661 inhibition of DPIV and DPIV-like enzyme activity was shown to represent an attractive approach for treating non-insulin-dependent diabetes mellitus (NIDDM).

The present invention provides a new use of DPIV-inhibitors for the prophylaxis and treatment of conditions mediated by inhibition of DPIV and DPIV-like enzymes, in particular the prophylaxis and treatment of multiple sclerosis, and pharmaceutical compositions e.g. useful in inhibiting DPIV and DPIV-like enzymes and a method of inhibiting said enzyme activity.

This invention relates to a method of treatment, in particular to a method for the prophylaxis and treatment of multiple sclerosis and to compounds and compositions for use in such method. Dipeptidyl peptidase IV (DPIV) is a post-proline (to a lesser extent post-alanine, post-serine or post-glycine) cleaving serine protease found in various tissues of the body including kidney, liver, and intestine.

It is known that DPIV-Inhibitors may be useful for the treatment of impaired glucose tolerance and diabetes mellitus (International Patent Application, Publication Number WO 99/61431, Pederson RA et al, Diabetes. 1998 Aug: 47(8):1253-8 and Pauly RP et al, Metabolism 1999 Mar; 48(3):385-9). In particular WO 99/61431 discloses DPIV-Inhibitors comprising an amino acid residue and a thiazolidine or pyrrolidine group, and salts thereof, especially L-*threo*-isoleucyl thiazolidine, L-*allo-*isoleucyl thiazolidine, L-*threo*-isoleucyl pyrrolidine, L-*allo*-isoleucyl thiazolidine, L-*allo-*isoleucyl pyrrolidine, and salts thereof.

Further examples of low molecular weight dipeptidyl peptidase IV inhibitors are agents such as tetrahydroisoquinolin-3-carboxamide derivatives, N-substituted 2-cyanopyroles and -pyrrolidines, N-(N'-substituted glycyl)-2-cyanopyrrolidines, N-(substituted glycyl)-thiazolidines, N-(substituted glycyl)-4-cyanothiazolidines, aminoacyl-borono-prolyl-inhibitors and cyclopropyl-fused pyrrolidines. Inhibitors of dipeptidyl peptidase IV are described in US 6,011,155; US 6,107,317; US 6,110,949; US 6,124,305; US 6,172,081; WO 99/61431, WO 99/67278. WO 99/67279, DE 198 34 591, WO 97140832, DE 196 16 486 C2, WO 98/19998, WO 00/07617, WO 99/38501, WO 99/46272, WO 99/38501, WO 01/68603, WO 01/40180, WO 01/81337, WO 01/81304, WO 01/55105, WO 02/02560 and WO 02/14271, the teachings of which are herein incorporated by reference in their entirety concerning these inhibitors, their uses, definition and their production.

The term DPIV-like enzymes relates to structurally and/or functionally DPIV/CD26-related enzyme proteins (Sedo & Malik, Dipeptidyl peptidase IV-like molecules: homologous proteins or homologous activities? Biochimica et Biophysica Acta 2001, 36506: 1-10). In essence, this small group of enzymes has evolved during evolution to release H-Xaa-Pro-Dipeptides and H-Xaa-Ala-Dipeptides from N-terminus of oligo- or polypeptides. They show the common feature, that they accomotate in the Pro-position also Al, Ser, Thr and other amino acids with small hydrophobic side-chains as, Gly or Val. The hydrolytic efficacy is ranked Pro>Ala» Ser, Thr » Gly, Val. Same proteins have been only available in such small quantities, that only the post-Pro or post-Ala cleavage could be established. While the proteins: DPIV, DP II, FAPα (Seprase), DP 6, DP 8 and DP 9 are structurally related and show a high sequence homology of , attraction is an extraordinary functional DPIV-like enzyme, characterized by a similar activity and inhibitory pattern.

Further DPIV-like enzymes are disclosed in WO 01/19866, WO 02/34900 and WO02/31134. WO 01/19866 discloses novel human dipeptidyl aminopeptidase 8 (DPP8) with structural und functional similarities to DPIV and fibroblast activation protein (FAP). WO 02/34900 discloses a novel dipeptidyl peptidase 9 (DPP9) with significant homology to the amino acid sequences of DPIV and DPP8. WO 02/31134 discloses three DPIV-like enzymes, DPRP1, DPRP2 and DPRP3. Sequence analysis revealed, that DPRP1 is identical to DPP8, as disclosed in WO 01/19866, that DPRP2 is identical to DPP9 and that DPRP3 is identical to KIAA1492 as disclosed in WO 02/04610.

Multiple sclerosis (MS) is a demyelinating disease of the central nervous system with a presumed autoimmune pathogenesis involving autoantigen-specific CD4⁺ T cells and cytokines (Rohowsky-Kochan, C, Molinaro, D, and Cook, SD. Cytokine secretion profile of myelin basic protein-specific T cells in multiple sclerosis. Multiple Sclerosis 6, 69-77. 2001.).

The degeneration underlying MS results from degradation of the myelin sheath, an electrically insulating fatty layer that surrounds nerve fibers and permits the rapid conduction of electrical signals. This loss of myelin can seriously impair the ability of neurons to conduct an electrical signal effectively. Symptoms will depend on where in the central nervous system (CNS) the myelin loss occurs and, thus, which nerve pathways become impaired.

The disease appears to be autoimmune in nature, i.e., the body's own immune system is responsible for the damage. The principal target of the autoimmune reaction appears to be Myelin Basic Protein (MBP), although other MS antigens have been proposed. In the early stages of the disease, a type of CNS cell called an oligodendrocyte can repair this damage and replace the lost myelin. However, these cells can also be destroyed in MS and so sufferers may lose the ability to repair the damage over time, which allows the disease to progress.

The mechanism of disease progression appears complex and several components of the immune system have been linked to the disease. While the underlying tissue damage appears to result predominantly from a T cell mediated response, antibodies against MS antigens are often present in the cerebrospinal fluid (CSF) and at active lesions. Antibodies are not normally present in the CSF and some disruption of the blood brain barrier (BBB), a protective membrane around the CNS, must also occur to allow antibodies, T cells and macrophages to enter the CSF. This change in the permeability of the BBB appears to be one of the defining events in the development of MS.

Activated macrophages secrete pro-inflammatory cytokines such as TNF-α and interferon-γ, leading to the production of destructive enzymes and free radicals. In addition to the complex nature of the immune response itself, the disease can also affect a number of targets. As well as the myelin sheath, other cells such as astrocytes and microglia can be attacked, forming discreet regions of damage known as plaques or lesions. The lesions are also known as scleroses (hence the name) and can occur in both the brain and the spinal cord. The sites of the lesions tend to be near blood vessels and are commonly found on the optic nerve, cerebellum, periventricular regions and spinal cord.

The initial mechanism for the onset of disease remains largely unknown and the number of active lesions at any given time is actually quite low. The heterogeneous nature of the disease and the number of clinical subtypes that this creates suggest that MS is probably a series of related conditions rather than one disease.

### Summary Of The Invention

The present invention provides a new use of DPIV-inhibitors. The compounds of formulas 1 to 2, and their corresponding pharmaceutically acceptable acid addition salt forms, are useful in treating conditions mediated by DPIV or DPIV-like enzymes, such as multiple sclerosis.

### Brief Description Of The Drawings

Figure 1: Illustrates the clinical course of experimental autoimmune encephalomyelitis (EAE). The effects of different dosages of isoleucyl thiazolidine fumarate on the clinical course of EAE in adult female Lewis rats were studied. Symbols represent means ± SEM of the mean clinical score per day. Two factor ANOVA for repeated measures revealed a significant interaction between the factors "treatment" "clinical score over time" indicating that during the initial acute phase of the disease between day 9-12(13), the DPIV inhibitors aggravated the disease while during between day 13-15 they improved or accelerated recovery from disease.
Figure 2: Illustrates the clinical course of experimental autoimmune encephalomyelitis (EAE) in isoleucyl thiazolidine fumarate treated rats split by days 10-15 post immunization. Separate one factor ANOVAs revealed significant disease-aggravating effect at the 1 mg dosage on day 11 and 12 p.i., while the 10mg dose significantly reduced clinical score at day 15 p.i. This indicates that the inhibitor initially tends to aggravate the disease while at later stages it results in an accelerated recovery from disease. Columns represent means ± SEM of the mean clinical score per day. Asterisks indicate significant post-hoc effects in the PLSD test with *<0.05 and **<0.001.
Figure 3: Illustrates the clinical course of experimental autoimmune encephalomyelitis (EAE). The effects of different dosages of isoleucyl thiazolidine fumarate during ongoing disease (days 5-15 p.i.) on the clinical course of EAE in adult male Lewis rats were studied. Symbols represent means ± SEM of the mean clinical score per day. Two factor ANOVA for repeated measures revealed significant main effects for the factor treatment and a significant interaction between the factors "treatment" and "clinical score over time" indicating that treatment significantly modulated the course of the disease and furthermore indicating that the different dosage act differentially. After initiation of treatment moderate dosages act proinflammatory and cause an "early peak" or aggravation of disease. During the acute phase of the disease between day 9-13, high dose of isoleucyl thiazolidine fumarate clearly improved the clinical course
Figure 4: Illustrates the clinical course of experimental autoimmune encephalomyelitis (EAE) in isoleucyl thiazolidine fumarate treated rats split by days 10-15 post immunization. Separate one factor ANOVAs revealed significant disease-aggravating effect at the 1 mg dosage on day 11 and 12 p.i., while the 10mg dose significantly reduced clinical score at day 15 p.i. This indicates that the inhibitor initially tends to aggravate the disease while at later stages it results in an accelerated recovery from disease. Columns represent means ± SEM of the mean clinical score per day. Asterisks indicate significant post-hoc effects in the PLSD test with *<0.05 and **<0.001.
Figure 5: Illustrates the clinical course of experimental autoimmune encephalomyelitis (EAE). The effects of different dosages of isoleucyl thiazolidine fumarate injected icv during ongoing disease (days 5-15 p.i.) on the clinical course of EAE in adult female Lewis rats were studied. Symbols represent means ± SEM of the mean clinical score per day and group. Two factor ANOVA for repeated measures revealed significant main effects for the factor treatment and a significant interaction between the factors "treatment" x "clinical score over time" indicating that treatment significantly modulated the course of the disease. All dosages of the DPIV-inhibitor exhibited ant-inflammatory effects with a delay of onset and an improvement of the clinical course.

### Detailed Description Of The Invention

The present invention relates to the area of dipeptidyl peptidase IV (DPIV) inhibition and, more particularly, to a new use of inhibitors of DPIV and DPIV-like enzyme activity for the treatment of multiple sclerosis, and pharmaceutical compositions containing said compounds.

In one illustrative embodiment, the present invention relates to dipeptide compounds and compounds analogous to dipeptide compounds that are formed from an amino acid and a thiazolidine or pyrrolidine group, and salts thereof, referred to hereinafter as dipeptide compounds.

Especially suitable for the purpose according to the invention are dipeptide compounds in which the amino acid is preferably selected from a natural amino acid, such as, for example, leucine, valine, glutamine, glutamic acid, proline, isoleucine, asparagines and aspartic acid.

The dipeptide compounds used according to the invention exhibit at a concentration (of dipeptide compounds) of 10 µM, indicated in Table 7, a reduction in the activity of dipeptidyl peptidase IV or DPIV-analogous enzyme activities of at least 10 %, especially of at least 40 %. Frequently a reduction in activity of at least 60 % or at least 70 % is also required. Preferred effectors may also exhibit a reduction in activity of a maximum of 20 % or 30 %.

Preferred compounds are L-*allo*-isoleucyl thiazolidine, L-*threo*-isoleucyl pyrrolidine and salts thereof, especially the fumaric salts, and L-*allo*-isoleucyl pyrrolidine and salts thereof. Especially preferred compounds are glutaminyl pyrrolidine and glutaminyl thiazolidine of formulas 1 and 2:

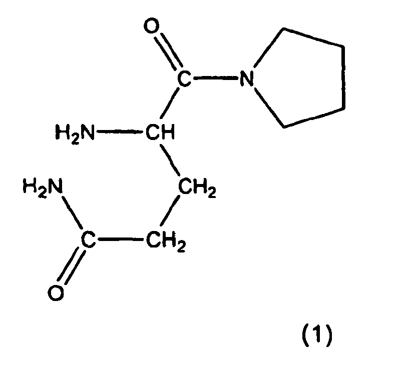

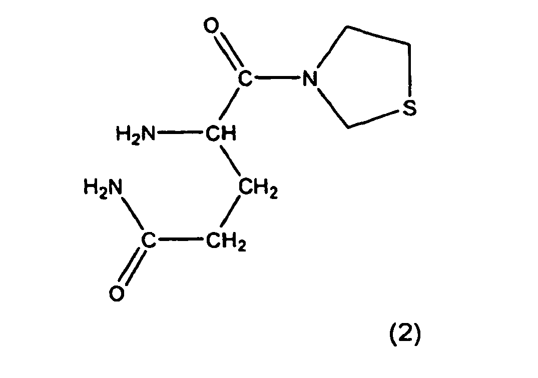

Further preferred compounds are given in Table 1.

The salts of the dipeptide compounds can be present in a molar ratio of dipeptide (-analogous) component to salt component of 1 : 1 or 2 : 1. Such a salt is, for example, (Ile-Thia)₂ fumaric acid.

**Table 1: Structures of further preferred dipeptide compounds**

| **Effector** |
|---|
| H-Asn-pyrrolidine |
| H-Asn-thiazolidine |
| H-Asp-pyrrolidine |
| H-Asp-thiazolidine |
| H-Asp(NHOH)-pyrrolidine |
| H-Asp(NHOH)-thiazolidine |
| H-Glu-pyrrolidine |
| H-Glu-thiazolidine |
| H-Glu(NHOH)-pyrrolidine |
| H-Glu(NHOH)-thiazolidine |
| H-His-pyrrolidine |
| H-His-thiazolidine |
| H-Pro-pyrrolidine |
| H-Pro-thiazolidine |
| H-Ile-azididine |
| H-Ile-pyrrolidine |
| H-L-allo-Ile-thiazolidine |
| H-Val-pyrrolidine |
| H-Val-thiazolidine |

Examples of amino acids throughout the claims and the description are:
aspartic acid (Asp), glutamic acid (Glu), arginine (Arg), lysine (Lys), histidine (His), glycine (Gly), serine (Ser) and cysteine (Cys), threonine (Thr), asparagine (Asn), glutamine (Gln), tyrosine (Tyr), alanine (Ala), proline (Pro), valine (Val), isoleucine (Ile), leucine (Leu), methionine (Met), phenylalanine (Phe), tryptophan (Trp), hydroxyproline (Hyp), beta-alanine (beta-Ala), 2-amino octanoic acid (Aoa), azetidine-(2)-carboxylic acid (Ace). Other amino acid substitutions for those encoded in the genetic code can also be included in peptide compounds within the scope of the invention and can be classified within this general scheme.

Proteinogenic amino acids are defined as natural protein-derived α-amino acids. Non-proteinogenic amino acids are defined as all other amino acids, which are not building blocks of common natural proteins.

The resulting peptides may be synthesized as the free C-terminal acid or as the C-terminal amide form. The free acid peptides or the amides may be varied by side chain modifications. Such side chain modifications are for instance, but not restricted to, homoserine formation, pyroglutamic acid formation, disulphide bond formation, deamidation of asparagine or glutamine residues, methylation, t-butylation, t-butyloxycarbonylation, 4-methylbenzylation, thioanysilation, thiocresylation, bencyloxymethylation, 4-nitrophenylation, bencyloxycarbonylation, 2-nitrobencoylation, 2-nitrosulphenylation, 4-toluenesulphonylation, pentafluorophenylation, diphenylmethylation, 2-chlorobenzyloxycarbonylation, 2,4,5-trichlorophenylation, 2-bromobenzyloxycarbonylation, 9-fluorenylmethyloxycarbonylation, triphenylmethylation, 2,2,5,7,8,-pentamethylchroman-6-sulphonylation, hydroxylation, oxidation of methionine, formylation, acetylation, anisylation, bencylation, bencoylation, trifluoroacetylation, carboxylation of aspartic acid or glutamic acid, phosphorylation, sulphation, cysteinylation, glycolysation with pentoses, deoxyhexoses, hexosamines, hexoses or N-acetylhexosamines, farnesylation, myristolysation, biotinylation, palmitoylation, stearoylation, geranylgeranylation, glutathionylation, 5'-adenosylation, ADP-ribosylation, modification with N-glycolylneuraminic acid, N-acetylneuraminic acid, pyridoxal phosphate, lipoic acid, 4'-phosphopantetheine, or N-hydroxysuccinimide.

Until the present invention by Applicants, known peptide substrates of the proline-specific serine protease dipeptidyl peptidase IV *in vitro* are the tripeptides Diprotin A (Ile-Pro-Ile), Diprotin B (Val-Pro-Leu) and Diprotin C (Val-Pro-Ile). Applicants have unexpectedly discovered that the compounds disclosed here act as substrates of dipeptidyl peptidase IV *in vivo* in a mammal and, in pharmacological doses, inhibit the physiological turnover of endogenous substrates by competitive catalysis.

Particularly preferred compounds of the present invention that are useful as modulators of dipeptidyl peptidase IV and DPIV - like enzymes include those compounds which show Kᵢ-values for DPIV binding, effectively in DPIV inhibition *in vivo* after i.v. and/or p.o. administration to Wistar rats

Throughout the description and the claims the expression "acyl" can denote a C₁₋₂₀ acyl residue, preferably a C₁₋₈ acyl residue and especially preferred a C₁₋₄ acyl residue, "cycloalkyl" can denote a C₃₋₁₂ cycloalkyl residue, preferably a C₄, C₅ or C₆ cycloalkyl residue, "carbocyclic" can denote a C₃₋₁₂ carbocyclic residue, preferably a C₄, C₅ or C₆ carbocyclic residue. "Heteroaryl" is defined as an aryl residue, wherein 1 to 4, preferably 1, 2 or 3 ring atoms are replaced by heteroatoms like N, S or O. "Heterocyclic" is defined as a cycloalkyl residue, wherein 1, 2 or 3 ring atoms are replaced by heteroatoms like N, S or O. "Peptides" are selected from dipeptides to decapeptides, preferred are dipeptides, tripeptides, tetrapeptides and pentapeptides. The amino acids for the formation of the "peptides" can be selected from the those listed above.

Throughout the description and the claims the expression "alkyl" can denote a C₁₋₅₀ alkyl group, preferably a C₆₋₃₀ alkyl group, especially a C₈₋₁₂ alkyl group; for example, an alkyl group may be a methyl, ethyl, propyl, isopropyl or butyl group. The expression "alk", for example in the expression "alkoxy", and the expression "alkan", for example in the expression "alkanoyl", are defined as for "alkyl"; aromatic compounds are preferably substituted or optionally unsubstituted phenyl, benzyl, naphthyl, biphenyl or anthracene groups, which preferably have at least 8 C atoms; the expression "alkenyl" can denote a C₂₋₁₀ alkenyl group, preferably a C₂₋₆ alkenyl group, which has the double bond(s) at any desired location and may be substituted or unsubstituted; the expression "alkynyl" can denote a C₂₋₁₀ alkynyl group, preferably a C₂₋₆ alkynyl group, which has the triple bond(s) at any desired location and may be substituted or unsubstituted; the expression "substituted" or substituent can denote any desired substitution by one or more, preferably one or two, alkyl, alkenyl, alkynyl, mono- or multi-valent acyl, alkanoyl, alkoxyalkanoyl or alkoxyalkyl groups; the aforementioned substituents may in turn have one or more (but preferably zero) alkyl, alkenyl, alkynyl, mono- or multi-valent acyl, alkanoyl, alkoxyalkanoyl or alkoxyalkyl groups as side groups; organic amines, amides, alcohols or acids, each having from 8 to 50 C atoms, preferably from 10 to 20 C atoms, can have the formulae (alkyl)₂N- or alkyl-NH-, -CO-N(alkyl)₂ or -CO-NH(alkyl), -alkyl-OH or -alkyl-COOH.

The compounds used in accordance with the invention can be present or used, respectively, in the form of racemates or in the form of enantiomerically pure compounds.

The compounds of the present invention can be converted into and used as acid addition salts, especially pharmaceutically acceptable acid addition salts. The pharmaceutically acceptable salt generally takes a form in which an amino acids basic side chain is protonated with an inorganic or organic acid. Representative organic or inorganic acids include hydrochloric, hydrobromic, perchloric, sulfuric, nitric, phosphoric, acetic, propionic, glycolic, lactic, succinic, maleic, fumaric, malic, tartaric, citric, benzoic, mandelic, methanesulfonic, hydroxyethanesulfonic, benzenesulfonic, oxalic, parnoic, 2-naphthalenesulfonic, p-toulenesulfonic, cyclohexanesulfamic, salicylic, saccharinic or trifluoroacetic acid. All pharmaceutically acceptable acid addition salt forms of the compounds of formulas 1 and 2 are intended to be embraced by the scope of this invention.

In view of the close relationship between the free compounds and the compounds in the form of their salts, whenever a compound is referred to in this context, a corresponding salt is also intended, provided such is possible or appropriate under the circumstances.

The present invention further includes within its scope prodrugs of the compounds of this invention. In general, such prodrugs will be functional derivatives of the compounds which are readily convertible *in vivo* into the desired therapeutically active compound. Thus, in these cases, the use of the present invention shall encompass the treatment of the various disorders described with prodrug versions of one or more of the claimed compounds, which convert to the above specified compound *in vivo* after administration to the subject. Conventional procedures for the selection and preparation of suitable prodrug derivatives are described, for example, in "Design of Prodrugs", ed. H. Bundgaard, Elsevier, 1985 and the patent applications DE 198 28 113 and DE 198 28 114, which are fully incorporated herein by reference.

Where the compounds or prodrugs according to this invention have at least one chiral center, they may accordingly exist as enantiomers. Where the compounds or prodrugs possess two or more chiral centers, they may additionally exist as diastereomers. It is to be understood that all such isomers and mixtures thereof are encompassed within the scope of the present invention. Furthermore, some of the crystalline forms of the compounds or prodrugs may exist as polymorphs and as such are intended to be included in the present invention. In addition, some of the compounds may form solvates with water (i.e. hydrates) or common organic solvents, and such solvates are also intended to be encompassed within the scope of this invention.

The compounds, including their salts, can also be obtained in the form of their hydrates, or include other solvents used for their crystallization.

As indicated above, the compounds and prodrugs of the present invention, and their corresponding pharmaceutically acceptable acid addition salt forms, are useful in inhibiting DPIV and DPIV - like enzyme activity. The ability of the compounds and prodrugs of the present invention, and their corresponding pharmaceutically acceptable acid addition salt forms to inhibit DPIV and DPIV - like enzyme activity may be demonstrated employing the DPIV activity assay for determination of the Kᵢ-values and the IC₅₀-values *in vitro,* as described in examples 7 and 8.

The ability of the compounds of the present invention, and their corresponding pharmaceutically acceptable acid addition salt forms to inhibit DPIV *in vivo* may be demonstrated by oral or intravasal administration to Wistar rats, as described in example 11. The compounds of the present invention inhibit DPIV activity *in vivo* after both, oral and intravasal administration to Wistar rats.

DPIV is present in a wide variety of mammalian organs and tissues e.g. the intestinal brush-border (Gutschmidt S. et al., "In situ" - measurements of protein contents in the brush border region along rat jejunal villi and their correlations with four enzyme activities. Histochemistry 1981, 72 (3), 467-79), exocrine epithelia, hepatocytes, renal tubuli, endothelia, myofibroblasts (Feller A.C. et al., A monoclonal antibody detecting dipeptidylpeptidase IV in human tissue. Virchows Arch. A. Pathol. Anat. Histopathol. 1986; 409 (2):263-73), nerve cells, lateral membranes of certain surface epithelia, e.g. Fallopian tube, uterus and vesicular gland, in the luminal cytoplasm of e.g., vesicular gland epithelium, and in mucous cells of Brunner's gland (Hartel S. et al., Dipeptidyl peptidase (DPP) IV in rat organs. Comparison of immunohistochemistry and activity histochemistry. Histochemistry 1988; 89 (2): 151-61), reproductive organs, e.g. cauda epididymis and ampulla, seminal vesicles and their secretions (Agrawal & Vanha-Perttula, Dipeptidyl peptidases in bovine reproductive organs and secretions. lnt. J. Androl. 1986, 9 (6): 435-52). In human serum, two molecular forms of dipeptidyl peptidase are present (Krepela E. et al., Demonstration of two molecular forms of dipeptidyl peptidase IV in normal human serum. Physiol. Bohemoslov. 1983, 32 (6): 486-96). The serum high molecular weight form of DPIV is expressed on the surface of activated T cells (Duke-Cohan J.S. et al., Serum high molecular weight dipeptidyl peptidase IV (CD26) is similar to a novel antigen DPPT-L released from activated T cells. J. Immunol. 1996, 156 (5): 1714-21).

The compounds and prodrugs of the present invention, and their corresponding pharmaceutically acceptable acid addition salt forms are able to inhibit DPIV *in vivo.* In one embodiment of the present invention, all molecular forms, homologues and epitopes of DPIV from all mammalian tissues and organs, also of those, which are undiscovered yet, are intended to be embraced by the scope of this invention.

Among the rare group of proline-specific proteases, DPIV was originally believed to be the only membrane-bound enzyme specific for proline as the penultimate residue at the amino-terminus of the polypeptide chain. However, other molecules, even structurally non-homologous with the DPIV but bearing corresponding enzyme activity, have been identified recently. DPIV-like enzymes, which are identified so far, are e.g. fibroblast activation protein α, dipeptidyl peptidase IV β, dipeptidyl aminopeptidase-like protein, N-acetylated α-linked acidic dipeptidase, quiescent cell proline dipeptidase, dipeptidyl peptidase II, attractin and dipeptidyl peptidase IV related protein (DPP 8), and are described in the review article by Sedo & Malik (Sedo & Malik, Dipeptidyl peptidase IV-like molecules: homologous proteins or homologous activities? Biochimica et Biophysica Acta 2001, 36506: 1-10). Further DPIV-like enzymes are disclosed in WO 01/19866, WO 02/34900 and WO02/31134. WO 01/19866 discloses novel human dipeptidyl aminopeptidase 8 (DPP8) with structural und functional similarities to DPIV and fibroblast activation protein (FAP). WO 02/34900 discloses a novel dipeptidyl peptidase 9 (DPP9) with significant homology to the amino acid sequences of DPIV and DPP8. WO 02/31134 discloses three DPIV-like enzymes, DPRP1, DPRP2 and DPRP3.

In another preferred embodiment of the present invention, all molecular forms, homologues and epitopes of proteins comprising DPIV-like enzyme activity, from all mammalian tissues and organs, are intended to be embraced by the scope of this invention.

The ability of the compounds and prodrugs of the present invention, and their corresponding pharmaceutically acceptable acid addition salt forms to inhibit DPIV-like enzymes may be demonstrated employing an enzyme activity assay for determination of the Kᵢ-values *in vitro* as described in example 5. The Kᵢ-values of the compounds of the present invention against porcine dipeptidyl peptidase II were exemplary determined as Kᵢ = 8.52*10⁻⁵ M ± 6.33*10⁻⁶ M for glutaminyl pyrrolidine and Kᵢ = 1.07*10⁻⁵ M ± 3.81*10⁻⁷ M for glutaminyl thiazolidine.

In another embodiment, the compounds and prodrugs of the present invention, and their corresponding pharmaceutically acceptable acid addition salt forms have only low, if no inhibitory activity against non-DPIV and non-DPIV - like proline specific enzymes. As described in example 6, with glutaminyl thiazolidine and glutaminyl pyrrolidine exemplarily, no inhibition of dipeptidyl peptidase I and prolyl oligopeptidase was found. Against prolidase, both compounds showed a marked lower efficacy compared to DPIV. The IC 50-values against prolidase were determined as IC 50 > 3mM for glutaminyl thiazolidine and as IC 50 = 3.4*10⁻⁴M ± 5.63*10⁻⁵ for glutaminyl pyrrolidine.

In view of their ability to inhibit DPIV and DPIV - like enzyme activity, the compounds of the present invention, and their corresponding pharmaceutically acceptable acid addition salt forms, are useful in treating conditions mediated by said enzyme activities. Based on the findings described in the examples of the present invention and in the literature, it can be shown that the compounds disclosed herein are useful in the treatment of conditions such as immune, autoimmune disorders or central nervous system disorders, selected from the group consisting of strokes, tumors, ischemia, Parkinson's disease, and migraines.

In a more preferred embodiment, the compounds and prodrugs of this invention, and their corresponding pharmaceutically acceptable acid addition salt forms, are useful for the treatment of multiple sclerosis. The ability of the compounds of the present invention, and their corresponding pharmaceutically acceptable acid addition salt forms, to alleviate the signs of multiple sclerosis can be determined employing the EAE rat model. The method is described in example 8.

The present invention therefore provides a method of preventing or treating a condition mediated by modulation of the DPIV or DPIV - like enzyme activity in a subject in need thereof which comprises administering any of the compounds of the present invention or pharmaceutical compositions thereof in a quantity and dosing regimen therapeutically effective to treat the condition. Additionally, the present invention includes the use of the compounds and progdrugs of this invention, and their corresponding pharmaceutically acceptable acid addition salt forms, for the preparation of a medicament for the prevention or treatment of a condition mediated by modulation of the DPIV activity in a subject. The compound may be administered to a patient by any conventional route of administration, including, but not limited to, intravenous, oral, subcutaneous, intramuscular, intradermal, parenteral and combinations thereof.

In a further illustrative embodiment, the present invention provides formulations for the compounds of formulas 1 to 2, and their corresponding pharmaceutically acceptable prodrugs and acid addition salt forms, in pharmaceutical compositions.

The term "subject" as used herein, refers to an animal, preferably a mammal, most preferably a human, who has been the object of treatment, observation or experiment.

The term "therapeutically effective amount" as used herein, means that amount of active compound or pharmaceutical agent that elicits the biological or medicinal response in a tissue system, animal or human, being sought by a researcher, veterinarian, medical doctor or other clinician, which includes alleviation of the symptoms of the disease or disorder being treated.

As used herein, the term "composition" is intended to encompass a product comprising the claimed compounds in the therapeutically effective amounts, as well as any product which results, directly or indirectly, from combinations of the claimed compounds.

To prepare the pharmaceutical compositions used in this invention, one or more compounds of formulas 1 to 2, or their corresponding pharmaceutically acceptable acid addition salt forms, as the active ingredient, is intimately admixed with a pharmaceutical carrier according to conventional pharmaceutical compounding techniques, which carrier may take a wide variety of forms depending of the form of preparation desired for administration, e.g., oral or parenteral such as intramuscular. In preparing the compositions in oral dosage form, any of the usual pharmaceutical media may be employed. Thus, for liquid oral preparations, such as for example, suspensions, elixirs and solutions, suitable carriers and additives may advantageously include water, glycols, oils, alcohols, flavoring agents, preservatives, coloring agents and the like; for solid oral preparations such as, for example, powders, capsules, gelcaps and tablets, suitable carriers and additives include starches, sugars, diluents, granulating agents, lubricants, binders, disintegrating agents and the like. Because of their ease in administration, tablets and capsules represent the most advantageous oral dosage unit form, in which case solid pharmaceutical carriers are employed. If desired, tablets may be sugar coated or enteric coated by standard techniques. For parenterals the carrier will usually comprise sterile water, through other ingredients, for example, for purposes such as aiding solubility or for preservation, may be included.

Injectable suspensions may also be prepared, in which case appropriate liquid carriers, suspending agents and the like may be employed. The pharmaceutical compositions herein will contain, per dosage unit, e.g., tablet, capsule, powder, injection, teaspoonful and the like, an amount of the active ingredient necessary to deliver an effective dose as described above. The pharmaceutical compositions herein will contain, per dosage unit, e.g., tablet, capsule, powder, injection, suppository, teaspoonful and the like, of from about 0.01 mg to about 1000 mg (preferably about 5 to about 500 mg) and may be given at a dosage of from about 0.1 to about 300 mg/kg bodyweight per day (preferably 1 to 50 mg/kg/day). The dosages, however, may be varied depending upon the requirement of the patients, the severity of the condition being treated and the compound being employed. The use of either daily administration or post-periodic dosing may be employed. Typically the dosage will be regulated by the physician based on the characteristics of the patient, his/her condition and the therapeutic effect desired.

Preferably these compositions are in unit dosage forms from such as tablets, pills, capsules, powders, granules, sterile parenteral solutions or suspensions, metered aerosol or liquid sprays, drops, ampoules, autoinjector devices or suppositories; for oral parenteral, intranasal, sublingual or rectal administration, or for administration by inhalation or insufflation. Alternatively, the composition may be presented in a form suitable for once-weekly or once-monthly administration; for example, an insoluble salt of the active compound, such as the decanoate salt, may be adapted to provide a depot preparation for intramuscular injection. For preparing solid compositions such as tablets, the principal active ingredient is ideally mixed with a pharmaceutical carrier, e.g. conventional tableting ingredients such as corn starch, lactose, sucrose, sorbitol, talc, stearic acid, magnesium stearate, dicalcium phosphate or gums, and other pharmaceutical diluents, e.g. water, to form a solid preformulation composition containing a homogeneous mixture of a compound of the present invention, or a pharmaceutically acceptable salt thereof. When referring to these preformulation compositions as homogeneous, it is meant that the active ingredient is ideally dispersed evenly throughout the composition so that the composition may be readily subdivided into equally effective dosage forms such as tablets, pills and capsules. This solid preformulation composition may then be subdivided into unit dosage forms of the type described above containing from about 0.01 to about 1000 mg, preferably from about 5 to about 500 mg of the active ingredient of the present invention.

The tablets or pills of the novel composition can be advantageously coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of materials can be used for such enteric layers or coatings, such materials including a number of polymeric acids with such materials as shellac, cetyl alcohol and cellulose acetate.

This liquid forms in which the novel compositions of the present invention may be advantageously incorporated for administration orally or by injection include, aqueous solutions, suitably flavored syrups, aqueous or oil suspensions, and flavored emulsions with edible oils such as cottonseed oil, sesame oil, coconut oil or peanut oil, as well as elixirs and similar pharmaceutical vehicles. Suitable dispersing or suspending agents for aqueous suspensions include synthetic and natural gums such as tragacanth, acacia, alginate, dextran, sodium carboxymethylcellulose, methylcellulose, polyvinylpyrrolidone or gelatin.

Where the processes for the preparation of the compounds according to the invention give rise to a mixture of stereoisomers, these isomers may be separated by conventional techniques such as preparative chromatography. The compounds may be prepared in racemic form, or individual enantiomers may be prepared either by enantiospecific synthesis or by resolution. The compounds may, for example, be resolved into their components enantiomers by standard techniques, such as the formation of diastereomeric pairs by salt formation with an optically active acid, such as (-)-di-p-toluoyl-d-tartaric acid and/or (+)-di-p-toluoyl-I-tartaric acid followed by fractional crystallization and regeneration of the free base. The compounds may also resolved by formation of diastereomeric esters or amides, followed by chromatographic separation and removal of the chiral auxiliary. Alternatively, the compounds may be resolved using a chiral HPLC column.

During any of the processes for preparation of the compounds of the present invention, it may be necessary and/or desirable to protect sensitive or reactive groups on any of the molecules concerned. This may be achieved by means of conventional protecting groups, such as those described in Protective Groups in Organic Chemistry, ed. J.F.W. McOmie, Plenum Press, 1973; and T.W. Greene & P.G.M. Wuts, Protective Groups in Organic Synthesis, John Wiley & Sons, 1991, fully incorporated herein by reference. The protecting groups may be removed at a convenient subsequent stage using methods known from the art.

The method of treating conditions modulated by dipeptidyl peptidase IV and DPIV - like enzymes described in the present invention may also be carried out using a pharmaceutical composition comprising one or more of the compounds as defined herein and a pharmaceutically acceptable carrier. The pharmaceutical composition may contain from about 0.01 mg to about 1000 mg, preferably about 5 to about 500 mg, of the compound(s), and may be constituted into any form suitable for the mode of administration selected. Carriers include necessary and inert pharmaceutical excipients, including, but not limited to, binders, suspending agents, lubricants, flavorants, sweeteners, preservatives, dyes, and coatings. Compositions suitable for oral administration include solid forms, such as pills, tablets, caplets, capsules (each including immediate release, timed release and sustained release formulations), granules, and powders, and liquid forms, such as solutions, syrups, elixirs, emulsions, and suspensions. Forms useful for parenteral administration include sterile solutions, emulsions and suspensions.

Advantageously, compounds of the present invention may be administered in a single daily dose, or the total daily dosage may be administered in divided doses of two, three or four times daily. Furthermore, compounds of the present invention can be administered in intranasal form via topical use of suitable intranasal vehicles, or via transdermal skin patches well known to those of ordinary skill in that art. To be administered in the form of transdermal delivery system, the dosage administration will, of course, be continuous rather than intermittent throughout the dosage regimen and dosage strength will need to be accordingly modified to obtain the desired therapeutic effects.

More preferably, for oral administration in the form of a tablet or capsule, the active drug component can be combined with an oral, non-toxic pharmaceutically acceptable inert carrier such as ethanol, glycerol, water and the like. Moreover, when desired or necessary, suitable binders; lubricants, disintegrating agents and coloring agents can also be incorporated into the mixture. Suitable binders include, without limitation, starch, gelatin, natural sugars such as glucose or betalactose, corn sweeteners, natural and synthetic gums such as acacia, tragacanth or sodium oleate, sodium stearate, magnesium stearate, sodium benzoate, sodium acetate, sodium chloride and the like. Disintegrators include, without limitation, starch, methyl cellulose, agar, bentonite, xanthan gum and other compounds known within the art.

The liquid forms are suitable in flavored suspending or dispersing agents such as the synthetic and natural gums, for example, tragacanth, acacia, methyl-cellulose and the like. For parenteral administration, sterile suspensions and solutions are desired. Isotonic preparations which generally contain suitable preservatives are employed when intravenous administration is desired.

The compound of the present invention can also be administered in the form of liposome delivery systems, such as small unilamellar vesicles, large unilamellar vesicles, and multilamellar vesicles. Liposomes can be formed from a variety of phospholipids, such as cholesterol, stearylamine or phosphatidylcholines using processes well described in the art.

Compounds of the present invention may also be coupled with soluble polymers as targetable drug carriers. Such polymers can include polyvinylpyrrolidone, pyran copolymer, polyhydroxypropylmethacrylamidephenol, polyhydroxyethylaspartamide-phenol, or polyethyl eneoxidepolyllysine substituted with palmitoyl residue. Furthermore, compounds of the present invention may be coupled to a class of biodegradable polymers useful in achieving controlled release of a drug, for example, polyactic acid, polyepsilon caprolactone, polyhydroxy butyeric acid, polyorthoesters, polyacetals, polydihydropyrans, polycyanoacrylates and cross-linked or amphipathic block copolymers of hydrogels.

Compounds of this invention may be administered in any of the foregoing compositions and according to dosage regimens established in the art whenever treatment of the addressed disorders is required.

The daily dosage of the products may be varied over a wide range from 0.01 to 1.000 mg per adult human per day. For oral administration, the compositions are preferably provided in the form of tablets containing, 0.01, 0.05, 0.1, 0.5, 1.0, 2.5, 5.0, 10.0, 15.0. 25.0, 50.0, 100, 150, 200, 250, 500 and 1000 milligrams of the active ingredient for the symptomatic adjustment of the dosage to the patient to be treated. An effective amount of the drug is ordinarily supplied at a dosage level of from about 0.1 mg/kg to about 300 mg/kg of body weight per day. Preferably, the range is from about 1 to about 50 mg/kg of body weight per day. The compounds may be administered on a regimen of 1 to 4 times per day.

Optimal dosages to be administered may be readily determined by those skilled in the art, and will vary with the particular compound used, the mode of administration, the strength of the preparation, bioavailability due to the mode of administration, and the advancement of disease condition. In addition, factors associated with the particular patient being treated, including patient age, weight, diet and time of administration, should generally be considered in adjusting dosages.

The compounds or compositions of the present invention may be taken before a meal, while taking a meal or after a meal.

### Examples

### Example 1: Synthesis Of Dipeptide Compounds

### 1.1 General synthesis of isoleucyl thiazolidine salt

The Boc-protected amino acid BOC-Ile-OH is placed in ethyl acetate and the batch is cooled to about - 5°C. N-Methylmorpholine is added dropwise, pivalic acid chloride (on a laboratory scale) or neohexanoyl chloride (on a pilot-plant scale) is added dropwise at constant temperature. The reaction is stirred for a few minutes for activation. N-Methylmorpholine (laboratory scale) and thiazolidine hydrochloride (laboratory scale) are added dropwise in succession, thiazolidine (pilot-plant scale) is added. Working-up in the laboratory is effected in conventional manner using salt solutions, on a pilot-plant scale the batch is purified with NaOH and CH₃COOH solutions.

The removal of the BOC protecting group is carried out using HCl/dioxane (laboratory scale) or H₂SO₄ (pilot-plant scale). In the laboratory the hydrochloride is crystallised from EtOH/ether.

On a pilot-plant scale the free amine is prepared by the addition of NaOH/NH₃. Fumaric acid is dissolved in hot ethanol, the free amine is added dropwise, and (Ile-Thia)² furmarate (M = 520.71 gmol⁻¹) precipitates. The analysis of isomers and enantiomers is carried out by electrophoresis.

### 1.2 Synthesis of glutaminyl pyrrolidine free base

### Acylation:

N-Benzyl-oxycarbonylglutamine (2.02 g, 7.21 mmol) was dissolved in 35 ml THF and brought to -15°C. Into that mixture CAIBE (isobutylchloroformiate) (0.937 ml, 7.21 mmol) and 4-methylmorpholine (0.795 ml, 7.21 mmol) where added and the solution was stirred for 15 min. The formation of the mixed anhydride was checked by TLC (eluent: CHCl₃/MeOH: 9/1). After warming to -10°C pyrrolidine (0.596 ml, 7.21 mmol) was added. The mixture was brought to room temperature and stirred overnight.

### Workup:

The sediment formed was filtered off and the solvent was evaporated. The resulting oil was taken up in ethylacetate (20 ml) and washed with a saturated solution of sodiumhydrogensulfate followed by a saturated solution of sodiumbicarbonate, water and brine. The organic layer was separated, dried and evaporated. The resulting product was checked for purity by TLC (eluent: CHCl₃/MeOH: 9/1)
Yield: 1.18 g, waxy solid

### Cleavage:

1.18 g of the resulting solid Z-protected compound was dissolved in 40 ml absolute ethanol. Into the solution ca. 20 mg Pd on charcoal (10%, FLUKA) was added and the suspension was shaken under a hydrogen atmosphere for 3h. The progress of the reaction was monitored by TLC (eluent: CHCl₃/MeOH: 9/1). After completion of the reaction the was removed to provide the free base.
Yield: 99%
The purity was checked by means of TLC: n-butanole/AcOH/water/ethylacetate: 1/1/1/1. R_{f} = 0.4. The identity of the reaction product was checked by NMR analysis.

### 1.3 Synthesis of glutaminyl thiazolidine hydrochloride

### Acylation:

N-t-Butyl-oxycarbonylglutamine (2.0 g, 8.12 mmol) was dissolved in 5ml THF and brought to -15°C. Into that mixture CAIBE (isobutylchloroformiate) (1,06 ml, 8.12 mmol) and 4-methylmorpholine (0.895 ml, 8.12 mmol) where added and the solution was stirred for 15 min. The formation of the mixed anhydride was checked by TLC (eluent: CHCl₃/MeOH: 9/1). After warming to -10°C another equivalent 4-methylmorpholine (0.895 ml, 8.12 mmol) and thiazolidine hydrochloride (1.02 g, 8.12 mmol) was added. The mixture was brought to room temperature and stirred overnight.

### Workup:

The sediment formed was filtered off and the solvent was evaporated. The resulting oil was taken up in chloroform (20 ml) and washed with a saturated solution of sodiumhydrogensulfate followed by a saturated solution of sodiumbicarbonate, water and brine. The organic layer was separated, dried and evaporated. The resulting product was checked for purity by TLC (eluent: CHCl₃/MeOH: 9/1)
Yield: 1.64 g, solid

### Cleavage:

640 mg of the resulting solid Boc-protected compound was dissolved in 3.1 ml ice cold HCl in dioxane (12.98 M, 20 equivalents) and left on ice. The progress of the reaction was monitored by TLC (eluent: CHCl₃/MeOH: 9/1). After completion of the reaction the solvent was removed and the resulting oil was taken up in methanole and evaporated again. After that the resulting oil was dried over phosphorous-V-oxide and triturated two times with diethylether. The purity was checked by HPLC.
Yield: 0.265 g
The purity was checked by HPLC. The identity of the reaction product was checked by NMR analysis.

### 1.4 Synthesis of Glutaminyl pyrrolidine hydrochloride

### Acylation:

N-t-Butyl-oxycarbonylglutamine (3.0 g, 12.18 mmol) was dissolved in 7ml THF and brought to -15°C. Into that mixture CAIBE (isobutylchloroformiate) (1,6 ml, 12.18 mmol) and 4-methylmorpholine (1.3 ml, 12.18 mmol) where added and the solution was stirred for 15 min. The formation of the mixed anhydride was checked by TLC (eluent: CHCl₃/MeOH: 9/1). After warming to -10°C 1 equivalent of pyrrolidine (1.0 ml, 12.18 mmol) was added. The mixture was brought to room temperature and stirred overnight.

### Workup:

The sediment formed was filtered off and the solvent was evaporated. The resulting oil was taken up in chloroform (20 ml) and washed with a saturated solution of sodiumhydrogensulfate followed by a saturated solution of sodiumbicarbonate, water and brine. The organic layer was separated, dried and evaporated. The resulting product was checked for purity by TLC (eluent: CHCl₃/MeOH: 9/1)
Yield: 2.7 g solid

### Cleavage:

2.7g of the resulting solid was dissolved in 13.0 ml ice cold HCl in dioxane (12.98 M, 20 equivalents) and left on ice. The progress of the reaction was monitored by TLC (eluent: CHCl₃/MeOH: 9/1). After completion of the reaction the solvent was removed and the resulting oil was taken up in methanole and evaporated again. After that the resulting oil was dried over phosphorous-V-oxide and triturated two times with diethylether.
Yield: 980 mg
The purity was checked by HPLC. The identity of the reaction product was checked by NMR analysis.

### Example 2: Chemical characterization of selected dipeptide compounds

### 2.1 Melting point determination

Melting points were determined on a Kofler heating platform microscope from Leica Aktiengesellschaft, the values are not corrected, or on a DSC apparatus (Heumann-Pharma).

### 2.2 Optical rotation

The rotation values were recorded at different wavelengths on a "Polarimeter 341" or higher, from the Perkin-Elmer company.

### 2.3 Measurement conditions for the mass spectroscopy

The mass spectra were recorded by means of electrospray ionisation (ESI) on an "API 165" or API 365" from the PE Sciex company. The operation is carried out using an approximate concentration of c = 10 µg/ml, the substance is taken up in MeOH/H₂O 50:50, 0.1 % HCO₂H, the infusion is effected using a spray pump (20 µl/min). The measurement were made in positive mode [M+H]⁺, the ESI voltage is U=5600V.

### 2.4. Results

### 2.4.1 Tests on isoleucyl thiazolodine fumarate (isomer)

| Substance | Mp (°C) | CE (min) | MS | [α]H₂O |
|---|---|---|---|---|
| L-threo-IT*F | 150^{DSC} | 160 | 203 | -10.7 |
| | | | | (405 nm) |
| D-threo-IT*F | 147 | 158 | 203 | not |
| | | | | determined |
| L-allo-IT*F | 145-6 | 154 | 203 | -4.58 |
| | | | | (380 nm) |
| D-allo-IT*F | 144-6 | 150 | 203 | 4.5 |
| | | | | (380 nm) |

| | | | | |
|---|---|---|---|---|
| IT*F = isoleucyl thiazolidine fumarate The NMR and HPLC data confirm the identity of the substance in question. | | | | |

### 2.4.2 Tests on other isoleucyl thiazolidine salts

| IT*salt | M (gmol⁻¹) | MP (°C) |
|---|---|---|
| succinate | 522.73 | 116 |
| tartrate | 352.41 | 122 |
| fumarate | 520.71 | 156 |
| hydrochloride | 238.77 | 169 |
| phosphate | 300.32 | 105 |

### Example 3: Kᵢ-determination

For Kᵢ determination of glutaminyl pyrrolidine and glutaminyl thiazolidine, dipeptidyl peptidase IV from porcine kidney with a specific activity against glycylprolyl-4-nitroaniline of 37.5 U/mg and an enzyme concentration of 1.41 mg/ml in the stock solution was used.

### Assay mixture:

100 µl test compound in a concentration range of 1*10⁻⁵ M - 1*10⁻⁸ M were admixed with 50 µl glycylprolyl-4-nitroaniline in different concentrations (0.4 mM, 0.2 mM, 0.1 mM, 0,05 mM) and 100 µl HEPES (40 mM, pH7.6; ion strength = 0.125). The assay mixture was pre-incubated at 30°C for 30 min. After pre-incubation, 20 µl DPIV (1:600 diluted) was added and measurement of yellow color development due to 4-nitroaniline release was performed at 30°C and = 405 nm for 10 min. using a plate reader (HTS7000 plus, Applied Biosystems, Weiterstadt, Germany).

The Kᵢ-values were calculated using Graphit version 4.0.13, 4.0.13 and 4.0.15 (Erithacus Software, Ltd, UK).

### 3.1 Results - Kᵢ values of DPIV inhibition

| **Compound** | **Ki [M]** |
|---|---|
| | |
| H-Asn-pyrrolidine | 1.20*10⁻⁵ |
| H-Asn-thiazolidine | 3.5*10⁻⁶ |
| H-Asp-pyrrolidine | 1.4*10⁻⁸ |
| H-Asp-thiazolidine | 2.9*10⁻⁶ |
| H-Asp(NHOH)-pyrrolidine | 1.3*10⁻⁵ |
| H-Asp(NHOH)-thiazolidine | 8.8*10⁻⁶ |
| H-Glu-pyrrolidine | 2.2*10⁻⁶ |
| H-Glu-thiazolidine | 6.1 *10⁻⁷ |
| H-Glu(NHOH)-pyrrolidine | 2.8*10⁻⁶ |
| H-Glu(NHOH)-thiazolidine | 1.7*10⁻⁶ |
| H-His-pyrrolidine | 3.5*10⁻⁶ |
| H-His-thiazolidine | 1.8*10⁻⁶ |
| H-Pro-pyrrolidine | 4.1 * 10⁻⁶ |
| H-Pro-thiazolidine | 1.2*10⁻⁶ |
| H-Ile-azididine | 3.1 *10⁻⁶ |
| H-Ile-pyrrolidine | 2.1*10⁻⁷ |
| H-L-*threo*-Ile-thiazolidine | 8.0*10⁻⁸ |
| H-L-*allo*-Ile-thiazolidine | 1.9*10⁻⁷ |
| D-*threo*-isoleucyl-thiazolidine-fumarate | no inhibition |
| D-*allo*-isoleucyl-thiazolidine-fumarate | no inhibition |
| H-L-*threo*-lle-thiazolidine-succinate | 5.1*10⁻⁸ |
| H-L-*threo*-lle-thiazolidine-tartrate | 8.3*10⁻⁸ |
| H-L-*threo*-lle-thiazolidine-fumarate | 8.3*10⁻⁸ |
| H-L-*threo*-lle-thiazolidine-hydrochloride | 7.2* 10⁻⁸ |
| H-L-*threo*-lle-thiazolidine-phosphate | 1.3*10⁻⁷ |
| H-Val-pyrrolidine | 4.8*10⁻⁷ |
| H-Val-thiazolidine | 2.7*10⁻⁷ |
| Diprotin A | 3.45*10⁻⁶ |
| Diprotin B | 2.24*10⁻⁵ |
| Gln-Pyrr | 2.26*10⁻⁶ |
| Gln-Thia | 1.21*10⁻⁶ |

### Example 4: Determination of IC₅₀-Values

100 µl inhibitor stock solution were mixed with 100 µl buffer (HEPES pH7.6) and 50 µl substrate (Gly-Pro-pNA, final concentration 0.4 mM) and preincubated at 30°C. Reaction was started by addition of 20 µl purified porcine DPIV. Formation of the product pNA was measured at 405 nm over 10 min using the HTS 7000Plus plate reader (Perkin Elmer) and slopes were calculated. The final inhibitor concentrations ranged between 1 mM and 30 nM. For calculation of IC50 GraFit 4.0.13 (Erithacus Software) was used.

### 4.1 Results - Determination of IC₅₀ values

| **Compound** | **IC50 [M]** |
|---|---|
| Ile-thiazolidine fumarate | 1.28*10⁻⁷ |
| Diprotin A | 4.69*10⁻⁶ |
| Diprotin B | 5.54*10⁻⁵ |
| Gln-Thia | 5.27*10⁻⁶ |
| Gln-Pyrr | 1.50*10⁻⁵ |

### Example 5: Inhibition Of DPIV-Like Enzymes - Dipeptidyl Peptidase II

DP II (3.4.14.2) releases N-terminal dipeptides from oligopeptides if the N-terminus is not protonated (McDonald, J.K., Ellis, S. & Reilly, T.J., 1966, J. Biol. Chem., 241, 1494-1501). Pro and Ala in P₁-position are preferred residues. The enzyme activity is described as DPIV-Iike activity, but DP II has an acidic pH-optimum. The enzyme used was purified from porcine kidney.

### Assay:

100 µl glutaminyl pyrrolidine or glutaminyl thiazolidine in an concentration range of 1*10⁻⁴ M - 5*10⁻⁸ M were admixed with 100 µl µl buffer solution (40 mM HEPES, pH7.6, 0.015% Brij, 1 mM DTT), 50 µl lysylalanylaminomethylcoumarine solution (5 mM) and 20 µl porcine DP II (250fold diluted in buffer solution). Fluorescence measurement was performed at 30°C and λ_{excitation} = 380 nm, λₑₘᵢₛₛᵢₒₙ = 465 nm for 25 min using a plate reader (HTS7000plus, Applied Biosystems, Weiterstadt, Germany). The Kᵢ-values were calculated using Graphit 4.0.15 (Erithacus Software, Ltd., UK) and were determined as Kᵢ = 8.52*10⁻⁵ M ± 6.33*10⁻⁶ M for glutaminyl pyrrolidine and Kᵢ = 1.07*10⁻⁵ M ± 3.81*10⁻⁷ M for glutaminyl thiazolidine.

### Example 6. Cross Reacting Enzymes

Glutaminyl pyrrolidine or glutaminyl thiazolidine were tested for their cross reacting potency against dipeptidyl peptidase I, prolyl oligopeptidase and Prolidase.

### Dipeptidyl peptidase I (DP I, cathepsin C):

DP I or cathepsin C is a lysosomal cysteine protease which cleaves off dipeptides from the N-terminus of their substrates (Gutman, H.R. & Fruton, J.S., 1948, J. Biol: Chem., 174, 851-858) . It is classified as a cysteine protease. The enzyme used was purchased from Qiagen (Qiagen GmbH, Hilden, Germany). In order to get a fully active enzyme, the enzyme was diluted 1000 fold in MES buffer pH5,6 (40 mM MES, 4 mM DTT, 4 mM KCI, 2 mM EDTA, 0.015% Brij) and pre-incubated for 30 min at 30°C.

### Assay:

50 µl glutaminyl pyrrolidine or glutaminyl thiazolidine in a concentration range of 1*10⁻⁵ M - 1*10⁻⁷ M were admixed with 110 µl buffer-enzyme-mixture. The assay mixture was pre-incubated at 30°C for 15 min. After pre-incubation, 100 µl histidylseryl-βnitroaniline (2*10⁻⁵M) was added and measurement of yellow color development due to β-nitroaniline release was performed at 30°C and λ_{excitation} = 380 nm, λₑₘᵢₛₛᵢₒₙ = 465 nm for 10 min., using a plate reader (HTS7000 plus, Applied Biosystems, Weiterstadt, Germany).

The IC ₅₀-values were calculated using Graphit 4.0.15 (Erithacus Software, Ltd., UK). No inhibition of the DP I enzyme activity by glutaminyl pyrrolidine or glutaminyl thiazolidine was found.

### Prolyl oligopeptidase (POP)

Prolyl oligopeptidase (EC 3.4.21.26) is a serine type endoprotease which cleaves off peptides at the N-terminal part of the Xaa-Pro bond (Walter, R., Shlank, H., Glass, J.D., Schwartz,I.L. & Kerenyi, T.D., 1971, Science, 173, 827-829). Substrates are peptides with a molecular weight up to 3000 Da. The enzyme used was a recombinant human prolyl oligopeptidase. Recombinant expression was performed in *E. coli* under standard conditions as described elsewhere in the state of the art.

### Assay.

100 µl glutaminyl pyrrolidine or glutaminyl thiazolidine in an concentration range of 1*10⁻⁴ M - 5*10⁻⁸ M were admixed with 100 µl µl buffer solution (40 mM HEPES, pH7.6, 0.015% Brij, 1 mM DTT) and 20 µl POP solution. The assay mixture was pre-incubated at 30 °C for 15 min. After pre-incubation, 50 µl glycylprolylprolyl-4-nitroaniline solution (0.29 mM) was added and measurement of yellow color development due to 4-nitroaniline release was performed at 30°C and λ = 405 nm for 10 min using a plate reader (sunrise, Tecan, Crailsheim, Germany). The IC ₅₀-values were calculated using Graphit 4.0.15 (Erithacus Software, Ltd., UK). No inhibition of POP activity by glutaminyl pyrrolidine or glutaminyl thiazolidine was found.

### Prolidase (X-Pro dipeptidase)

Prolidase (EC 3.4.13.9) was first described by Bergmann & Fruton (Bergmann, M. & Fruton, JS, 1937, J. Biol. Chem. 189-202). Prolidase releases the N-terminal amino acid from Xaa-Pro dipeptides and has a pH optimum between 6 and 9.

Prolidase from porcine kidney (ICN Biomedicals, Eschwege, Germany). was solved (1 mg/ml) in assay buffer (20mM NH₄(CH₃COO)₂, 3mM MnCl₂, pH 7.6). In order to get a fully active enzyme the solution was incubated for 60 min at room temperature.

### Assay:

450 µl glutaminyl pyrrolidine or glutaminyl thiazolidine in an concentration range of 5*10⁻³ M - 5*10⁻⁷ M were admixed with 500 µl buffer solution (20mM NH₄(CH₃COO)₂, pH 7.6) and 250 µl Ile-Pro-OH (0.5mM in the assay mixture). The assay mixture was pre-incubated at 30 °C for 5 min. After pre-incubation, 75 µl Prolidase (1:10 diluted in assay buffer) were added and measurement was performed at 30°C and λ = 220 nm for 20 min using a UV/Vis photometer, UV1 (Thermo Spectronic, Cambridge, UK).

The IC ₅₀-values were calculated using Graphit 4.0.15 (Erithacus Software, Ltd., UK). They were determined as IC ₅₀ > 3mM for glutaminyl thiazolidine and as IC ₅₀ = 3.4* 10⁻⁴M ± 5.63*10⁻⁵ for glutaminyl pyrrolidine.

### Example 7: Determination Of DPIV Inhibiting Activity After Intravasal And Oral Administration To Wistar Rats

### Animals

Male Wistar rats (Shoe: Wist(Sho)) with a body weight ranging between 250 and 350 g were purchased from Tierzucht Schönwalde (Schönwalde, Germany).

### Housing conditions

Animals were single-caged under conventional conditions with controlled temperature (22±2 °C) on a 12/12 hours light/dark cycle (light on at 06:00 AM). Standard pelleted chow (ssniff^{®} Soest, Germany) and tap water acidified with HCl were allowed ad libitum.

### Catheter insertion into carotid artery

After ≥one week of adaptation at the housing conditions, catheters were implanted into the carotid artery of Wistar rats under general anaesthesia (i.p. injection of 0.25 ml/kg b.w. Rompun^{®} [2 %], BayerVital, Germany and 0.5 ml/kg b.w. Ketamin 10, Atarost GmbH & Co., Twistringen, Germany). The animals were allowed to recover for one week. The catheters were flushed with heparin-saline (100 IU/ml) three times per week. In case of catheter dysfunction, a second catheter was inserted into the contra-lateral carotid artery of the respective rat. After one week of recovery from surgery, this animal was reintegrated into the study. In case of dysfunction of the second catheter, the animal was withdrawn from the study. A new animal was recruited and the experiments were continued in the planned sequence, beginning at least 7 days after catheter implantation.

### Experimental design

Rats with intact catheter function were administered placebo (1 ml saline, 0.154 mol/l) or test compound via the oral and the intra-vasal (intra-arterial) route. After overnight fasting, 100 µl samples of heparinised arterial blood were collected at -30, -5, and 0 min. The test substance was dissolved freshly in 1.0 ml saline (0.154 mol/l) and was administered at 0 min either orally via a feeding tube (75 mm; Fine Science Tools, Heidelberg, Germany) or via the intra-vasal route. In the case of oral administration, an additional volume of 1 ml saline was injected into the arterial catheter. In the case of intra-arterial administration, the catheter was immediately flushed with 30 µl saline and an additional 1 ml of saline was given orally via the feeding tube.

After application of placebo or the test substances, arterial blood samples were taken at 2.5, 5, 7.5, 10, 15, 20, 40, 60 and 120 min from the carotid catheter of the conscious unrestrained rats. All blood samples were collected into ice cooled Eppendorf tubes (Eppendorf-Netheler-Hinz, Hamburg, Germany) filled with 10 µl 1 M sodium citrate buffer (pH 3.0) for plasma DPIV activity measurement. Eppendorf tubes were centrifuged immediately (12000 rpm for 2 min, Hettich Zentrifuge EBA 12, Tuttlingen; Germany): The plasma fractions were stored on ice until analysis or were frozen at -20 °C until analysis. All plasma samples were labelled with the following data:
• Code number
• Animal Number
• Date of sampling
• Time of sampling

### Analytical Methods

The assay mixture for determination of plasma DPIV activity consisted of 80 µl reagent and 20 µl plasma sample. Kinetic measurement of the formation of the yellow product 4-nitroaniline from the substrate glycylprolyl-4-nitroaniline was performed at 390 nm for 1 min at 30 °C after 2 min pre-incubation at the same temperature. The DPIV activity was expressed in mU/ml.

### Statistical methods

Statistical evaluations and graphics were performed with PRISM^{®} 3.02 (GraphPad Software, Inc.). All parameters were analysed in a descriptive manner including mean and SD.

### 7. Results - in vivo DPIV-inhibition at tₘₐₓ

| STRUCTURE | **Dose (mg/kg)** | **i.v. (%)** | **p.o. (%)** |
|---|---|---|---|
| Gln-Pyrr | 100 | 80 | 67 |
| Gln-Thia | 100 | 88 | 71 |
| Diprotin A | 100 | 73 | no inhibition |
| Diprotin B | 100 | 50 | no inhibition |
| Ile-cyclopentyl-ketone | 100 | 34 | 15 |

### Example 8 Efficacy Of Dipeptidyl-Peptidase IV (DPIV; CD26) Inhibitors In Models For Multiple Sclerosis

### 8.1 Experiment 1: i. p. treatment (0-30 mg/kg b. w. isoleucyl thiazolidine fumarate/day) from day 1-15. Scores until day 15 p.i.

### Materials and methods

### Animals

For the first experiment female inbred Lewis rats (n = 50) with a mean body weight of 190 ± 6g, were obtained from Charles River (Bad Sulzfeld, Germany). Animals were randomly assigned to the different experimental conditions. Rats were housed four per cage under a 12:12 h light: dark cycle (lights off at 18:00 h) and at constant temperature (24°C) in a specific pathogen free air-conditioned colony room with food (Altromin lab chow pellets) and tap water available ad lib. The animals underwent routine cage maintenance once a week.

### Induction of EAE

Guinea pig MBP (50 µg/rat) was emulsified in Complete Freund's adjuvant (CFA) containing heat-killed *Mycobacterium tuberculosis* (H37Ra; 225 µg/rat) and injected s.c. at the base of the tail in a total volume of 100 µl (12). CFA (Sigma) and heat-killed *Mycobacterium tuberculosis* (H37Ra) were purchased from Life Technologies, Inc. (Rockville, MD). Clinical disease was scored on the following scale: 0.5: partial loss of tail tone; 1.0: complete tail atony; 2.0: hind limb weakness; 2.5: hind limb paralysis of one leg; 3.0: hind limb paralysis of both legs; 3.5: limb paralysis of three legs; 4.0: quadriplegia and moribund status; 5.0: death due to EAE. Experiments were terminated on day 15 post immunization when most of the animals were about 48h after maximal clinical score. The incidence of adjuvant-induced arthritis was remarkably low (*n=0* in the 1^{st} experiment) using the base of the tail as an immunization site. In general, animals exhibiting signs of arthritis or not developing any signs of EAE or showing signs of peritonitis on autopsy were excluded from further analysis. In the first experiment no signs of arthritis and no sudden deaths and/or peritonitis due to repeated ip-injection were found. Two animals in the 1 mg/kg-treatment-group did not develop clear signs of EAE (score > 1) and therefore were excluded from further analysis although this eventually might reflect a treatment effect. Immunization at the base of tail might cause acute inflammation, which in turn might affect the tone of the tail during the first days post immunization.

### Statistical analysis

Comparison over time between the clinical course of EAE of the different treatment conditions was conducted using a two factorial analysis of variance (ANOVA) for repeated measures. Sums of clinical scores of EAE, onset and peak of disease were calculated on the basis of the clinical scores per day and analyzed using a one factorial ANOVA and post hoc Fisher's PLSD tests, if appropriate. All data are presented as means ± SEM.

### Results

### Effect of daily injections of isoleucyl thiazolidine fumarate on clinical course of EAE in Lewis rats

The clinical course of the EAE is illustrated in Figure 1. Two factor ANOVA for repeated measures (treatment x clinical scores over time) revealed a significant interaction of the two factors (F(4,56) = 1.7; p = 0.001) indicating a differential course of the disease. This interaction is most probably due to the fact that inhibitor treatment aggravated the initial course of EAE while it improved recovery from disease.

Separate one factor ANOVAs split by day revealed significant (day 11: F(4,43) = 2.8; p < 0.05; day 12: F(4,43) = 3.0; p < 0.05) disease-aggravating effects at the 1mg dosage on days 11 and 12 post immunization (p.i.), while the 10mg dose significantly (F(4,43) = 4.8; p < 0.001) reduced clinical score at day 15 p.i. This again indicates that the inhibitor initially tends to aggravate the disease while at later stages it results in an accelerated recovery from disease. Further analysis of key parameter derived from the clinical course demonstrated that inhibitor treatment at the 10mg/kg dosage shortened the latency until onset of disease for about 1 day.

### Conclusion

Daily intraperitoneal treatment with the DPIV-inhibitor isoleucyl thiazolidine fumarate over a wide range of dosages in Lewis rats for a period of 15 days post immunization with MBP in CFA showed that the drug initially aggravates and during recovery phase improves the clinical course of the disease. It is possible that initial pro-inflammatory effects during acute disease may switch into anti-inflammatory or other clinics improving effects after reaching peak of disease. These late effects may overall be beneficial. Further experiments will test this hypothesis by comparing "early" vs. "late" or "induction" vs. "ongoing" disease effects.

### 8.2 Experiment 2: i.p. treatment (0-30 mglkg b.w. isoleucyl thiazolidine fumarate/day) from day 5-15. Scores until day 21 p.i.

### Materials and methods

### Animals

For the second experiment, male inbred Lewis rats (n = 50) with a mean body weight of 230 ± 12g, were obtained from Charles River (Bad Sulzfeld, Germany). Animals were randomly assigned to the different experimental conditions. Rats were housed four per cage under a 12:12 h light: dark cycle (lights off at 18:00 h) and at constant temperature (24°C) in a specific pathogen free air-conditioned colony room with food (Altromin lab chow pellets) and tap water available ad lib. The animals underwent routine cage maintenance once a week.

### Induction of EAE

Guinea pig MBP (50 µg/rat) was emulsified in Complete Freund's adjuvant (CFA) containing heat-killed *Mycobacterium tuberculosis* (H37Ra; 225 µg/rat) and injected s.c. at the base of the tail in a total volume of 100 µl (12). CFA (Sigma) and heat-killed *Mycobacterium tuberculosis* (H37Ra) were purchased from Life Technologies, Inc. (Rockville, MD). Clinical disease was scored on the following scale: 0.5: partial loss of tail tone; 1.0: complete tail atony; 2.0: hind limb weakness; 2.5: hind limb paralysis of one leg; 3.0: hind limb paralysis of both legs; 3.5: limb paralysis of three legs; 4.0: quadriplegia and moribund status; 5.0: death due to EAE. Experiments were terminated on day 21 post immunization (p.i.).

### General observations during EAE in the second experiment

The incidence of adjuvant-induced arthritis was remarkably low (*n*=0 in the 2^{nd} experiment) using the base of the tail as an immunization site. In the second experiment no signs of arthritis were found. Surprisingly, and similar in comparison with the first experiment, four animals in the 1mg/kg-treatment-group did not develop clear signs of EAE (score > 1). This time, these animals remained in the analysis, because this finding is probably due to the treatment. In this experiment a phenomenon called "disease dissociation" was observed. In several animals there was a clear tonus of the tail associated with a clear weakness of the hind limbs. The animals were scored maximal, i.e. 2.

### Statistical analysis

Comparison over time between the clinical course of EAE of the different treatment conditions was conducted using a two factorial analysis of variance (ANOVA) for repeated measures. Sums of clinical scores of EAE, onset and peak of disease were calculated on the basis of the clinical scores per day and analyzed using a one factorial ANOVA and post hoc Fisher's PLSD tests, if appropriate. All data are presented as means ± SEM.

### Results

### Effect of daily injections of isoleucyl thiazolidine fumarate during ongoing EAE in male Lewis rats

The clinical course of the EAE is illustrated in Figure 3. Two factor ANOVA for repeated measures (treatment x clinical scores over time) revealed a significant effect for the factor treatment (F(4,45) = 4.3; p = 0.0048) and a significant interaction of the two factors (F(4,45) = 3.5; p < 0.0001) indicating a differential course of the disease. This interaction is most probably due to the fact that low dose inhibitor treatment aggravated or even "induced" an early peak in the initial course of EAE while high dose improved cleary inhibited the acute phase of the disease. Separate one factor ANOVAs split by day revealed significant disease aggravating effects in two peaks of the disease. The 3mg and 10mg dosages induced a significant first peak of disease activity directly after initiation of treatment on days 6-9 (see figure 3; day 6: F(4,41) = 6.7; p = 0.0003; day 7: F(4,41) = 13.4; p < 0.0001; day 8: F(4,41) = 10.0; p < 0.0001; day 9: F(4,41) = 6.0; p = 0.0007). In the second peak, the "classical acute EAE", which was more severe than the first one, the 1mg dose aggravate the clinical scores while the high dosage of 30mg/kg exerted significant delaying and ameliorating effects (see figure 4; day 10: F(4,41) = 16; p < 0.0001; day 11: F(4,41) = 13.5; p < 0.0001; day 12: F(4,41) = 8.0; p < 0.0001; day 13: F(4,43) = 3.4; p = 0.017). This indicates proinflammatory effects of low dose inhibitor treatment while high dose inhibitior acts protective or anti-inflammatory-like in this model of MS. Further analysis of key parameters derived from the clinical course demonstrated that inhibitor treatment at the 1 mg/kg dosage exerts pro-inflammatory effects while 30mg/kg mediated protective-like or anti-inflammatory effects with a delay in the onset.

| Clinical scores of EAE in male Lewis rats treated from days 5-15(21) p.i. | | | | | |
|---|---|---|---|---|---|
| | | | | | |
| | isoleucyl thiazolidine fumarate | | | | |
| Scores | 0mg/kg | 1mglkg | 3mg/kg | 10mg/kg | 30mg/kg |
| Onset of disease | 11.8 | 10.3* | 12.0 | 12.1 1 | 14.0*** |
| (1st day of score >1) | | | | | |
| Maximal score | 2.6 | 2.8 | 3.0 | 2.1 | 2.0* |
| Sum of scores | 10.2 | 14.2* | 12.0 | 10.8 | 5.6** |
| Average score | 0.68 | 0.96* | 0.8 | 0.72 | 0.37** |

The various key parameters derived from EAE scores significantly differ in rats treated with different dosages of isoleucyl thiazolidine fumarate from days 5-15. Proinflammatory effects of 1 mg and anti-inflammatory effects of 30mg/kg are evident. Data represent means. *p<0.05, **p<0.01, ***p<0.0001 vs. control.

### Conclusion

This experiment investigated the effects of daily intraperitoneal treatment with the DPIV-Inhibitor isoleucyl thiazolidine fumarate over a wide range of dosages in male Lewis rats for 10 days with the treatment being initiated at day 5 post immunization with MBP in CFA. Treatment caused a significant dose-dependent and bimodal treatment effect in this design and model for MS. Low dose (1 mg) of the drug initially aggravates the acute phase of EAE. Moderate dose of isoleucyl thiazolidine fumarate (3 and 10mg) induces an early "first peak" of disease directly after initiation of disease, suggestive for direct pro-inflammatory effects *in vivo.* High dose of the drug (30mg) exerts clearly a potent anti-inflammatory effect and a delay in the onset of disease.

### 8.3 Experiment 3: i. v. treatment during ongoing disease; 0-50 nmol isoleucyl thiazolidine fumarate/5 µl per rat daily from days 5-15)

### Materials and method

### Animals

For the 3^{rd} experiment female inbred Lewis rats (*n* = 50) with a mean body weight of 201 ± 5g, were obtained from Charles River, Germany. Animals were randomly assigned to the different experimental conditions. Rats were housed four per cage under a 12:12 h light: dark cycle (lights off at 18:00 h) and at constant temperature (24°C) in a specific pathogen free air-conditioned colony room with food (Altromin lab chow pellets) and tap water available ad lib. The animals underwent routine cage maintenance once a week.

### Surgery and i.c.v. application

Under ketamine/xylasine (100/5 mg/kg, i.p.) anesthesia, the rats were fixed in a Kopf stereotaxic frame and implanted with cannulae (Plastic One, Inc., Roanoke, VA, USA) above the lateral ventricle. (coordinates: A:-0.7mm caudal, L:1.4mm lateral to bregma; and V:3.2mm ventral to the skull surface; tooth bar +3.0 above ear bars) using standard stereotaxic procedures as described in detail elsewhere. After a 4-day recovery period, rats were habituated to experimental handling by daily sham injections for another three days. On day seven after i.c.v. cannula implantation, EAE was induced

### Induction of EAE

Guinea pig MBP (50 µg/rat) was emulsified in Complete Freund's adjuvant (CFA) containing heat-killed *Mycobacterium tuberculosis* (H37Ra; 225 µg/rat) and injected s.c. at the base of the tail in a total volume of 100 µl (12). CFA (Sigma) and heat-killed *Mycobacterium tuberculosis* (H37Ra) were purchased from Life Technologies, Inc. (Rockville, MD). Clinical disease was scored on the following scale: 0.5: partial loss of tail tone; 1.0: complete tail atony; 1.5: complete tail atony and weakness of one hind limb; 2.0: weakness of both hind limbs; 2.5: hind limb weakness and paralysis of one hind leg; 3.0: paralysis of both hind legs; 3.5: limb paralysis of three legs; 4.0: quadriplegia and moribund status; 5.0: death due to EAE. Experiments were terminated on day 15 post immunization (p.i.).

### General observations during EAE in the 3^{rd} experiment

In the 3*^{rd}* experiment no signs of arthritis were found. One animal of the vehicle control group lost the icv-cannula and was excluded from further analysis. All other animals tolerated the treatment without any signs of aversiveness or sickness.

### Statistical analysis

Comparison over time between the clinical courses of EAE of the different treatment conditions was conducted using a two factorial analysis of variance (ANOVA) for repeated measures. Sums of clinical scores of EAE, onset and peak of disease were calculated on the basis of the clinical scores per day and analyzed using a one factorial ANOVA and post hoc Fisher's PLSD tests, if appropriate. All data are presented as means ± SEM.

### Results

### Effect of daily icv injections of isoleucyl thiazolidine fumarate during ongoing EAE in female Lewis rats

The clinical course of the EAE is illustrated in Figure 5. Two factor ANOVA for repeated measures (treatment x clinical scores over time) revealed a significant effect for the factor treatment (F(4,56) = 3.0; p = 0.03) and a significant interaction of the two factors (F(4,56) = 2.1; p < 0.0001) indicating a differential course of the disease. This interaction is most probably due to the fact that all dosages of the compound delayed and/or ameliorated the clinical course of EAE.

Further analysis of key parameter derived from the clinical course demonstrated that inhibitor treatment exerted anti-inflammatory effects on all parameters investigated

| Clinical score of EAE in female Lewis rats treated icv from day 5-15 p.i. | | | | | |
|---|---|---|---|---|---|
| | | | | | |
| | isoleucyl thiazolidine fumarate | | | | |
| Scores | 0 nmol | 0.5 nmol | 5 nmol | 10 nmol | 50 nmol |
| Onset of disease (1 st day of score 1) | 9.6 | 10.5 | 11.2* | 12.5** | 13.5** |
| Maximal score | 2.5 | 2.75 | 2.0 | 2.0 | 1.6* |
| Sum of scores | 11.5 | 10.6 | 6.5* | 6.7* | 4.9** |
| Average score | 0.76 | 0.7 | 0.44* | 0.45* | 0.31** |
| The various key parameters derived from EAE scores significantly differ in rats treated with different dosages of isoleucyl thiazolidine fumarate from days 5-15. Dose-dependent and potent anti-inflammatory effects of 5-50 nmol are evident. Data represent means. *p<0.05, **P<0.01. **p<0.0001 vs. control. | | | | | |

### Conclusion

This experiment investigated the effects of daily icv treatment with the DPIV-Inhibitor isoleucyl thiazolidine fumarate over a wide range of dosages in female Lewis rats for 10 days with the treatment being conducted from day 5-15 post immunization with MBP in CFA. This design was aimed at investigating the effects of the drug on the cellular components constituting the local inflammation within the CNS during EAE. Icv treatment with 5-50nmol dosages of isoleucyl thiazolidine fumarate caused a dose-dependent anti-inflammatory effect. These findings showpotent anti-inflammatory effect.

## Claims

1. Use of at least one inhibitor of dipeptidyl peptidase IV (DPIV) or DPIV-like enzyme activity for the preparation of a pharmaceutical composition for the treatment of multiple sclerosis, wherein the inhibitor is a dipeptide compound selected from the group consisting of L-*threo*-isoleucyl pyrrolidine, L-*allo*-isoleucyl thiazolidine, L-*allo-*isoleucyl pyrrolidine, L-*threo*-isoleucyl thiazolidine, L-glutaminyl thiazolidine, L-glutaminyl pyrrolidine, L-glutamic acid thiazolidine, L-glutamic acid pyrrolidine, H-Asn-pyrrolidine, H-Asn-thiazolidine, H-Asp-pyrrolidine, H-Asp-thiazolidine, H-Asp(NHOH)-pyrrolidine, H-Asp(NHOH)-thiazolidine, H-Glu(NHOH)-pyrrolidine, H-Glu(NHOH)-thiazolidine, H-His-pyrrolidine, H-His-thiazolidine, H-Pro-pyrrolidine, H-Pro-thiazolidine, H-Ile-azetidine, H-Val-pyrrolidine, H-Val-thiazolidine, and salts and/or solvates thereof.

2. The use according to claim 1, wherein the salt is formed from an inorganic or organic acid selected from the group consisting of hydrochloric, hydrobromic, perchloric, sulfuric, nitric, phosphoric, acetic, propionic, glycolic, lactic, succinic, maleic, fumaric, malic, tartaric, citric, benzoic, mandelic, methanesulfonic, hydroxyethanesulfonic, benzenesulfonic, oxalic, pamoic, 2-naphthalenesulfonic, p-toulenesulfonic, cyclohexanesulfamic, salicylic, saccharinic or trifluoroacetic acid.

3. The use according to claims 1 or 2, wherein the dipeptidyl peptidase IV-like enzyme is selected from the group consisting of fibroblast activation protein α, dipeptidyl peptidase IV β, dipeptidyl aminopeptidase-like protein, N-acetylated α-linked acidic dipeptidase, quiescent cell proline dipeptidase, dipeptidyl peptidase II, attractin, dipeptidyl peptidase IV related protein (DPP 8), dipeptidyl peptidase 9 (DPP 9), KIAA1492, DPRP1, DPRP2 and DPRP3.

4. The use according to any one of the preceding claims, wherein said pharmaceutical composition comprises a pharmaceutically acceptable carrier or diluent and a therapeutically effective amount of said inhibitor or a pharmaceutically acceptable acid addition salt and/or solvate thereof.

5. The use according to any one of the preceding claims, wherein the inhibitor is used in combination with a pharmaceutically acceptable carrier and/or diluent.

## Patentansprüche

1. Verwendung von mindestens einem Inhibitor der Dipeptidyl Peptidase IV (DPIV) oder DPIV-ähnlicher Enzymaktivität zur Herstellung einer pharmazeutischen Zusammensetzung für die Behandlung von Multipler Sklerose, wobei der Inhibitor ein Dipeptidanalogon, ausgewählt aus der Gruppe bestehend aus L-threo-Isoleucyl Pyrrolidin, L-allo-Isoleucyl Thiazolidin, L-allo-Isoleucyl Pyrrolidin, L-threo-Isoleucyl Thiazoliodin, L-Glutaminyl Thiazolidin, L-Glutaminyl Pyrrolidin, L-Glutaminsäure Pyrrolidin, L-Glutaminsäure Thiazolidin, H-Asn-Pyrrolidin, H-Asn-Thiazoldin, H-Asp-Pyrrolidin, H-Asp-Thiazoldin, H-Asp(NHOH)-Pyrrolidin, H-Asp(NHOH)-Thiazoldin, H-Glu(NHOH)-Pyrrolidin, H-Glu(NHOH)-Thiazoldin, H-His-Pyrrolidin, H-His-Thiazoldin, H-Pro-Pyrrolidin, H-Pro-Thiazoldin, H-Ile-Azetidin, H-Val-Pyrrolidin, H-Val-Thiazoldin, und deren Salzen und/oder Solvaten ist.

2. Die Verwendung nach Anspruch 1, wobei das Salz aus einer anorganischen oder organischen Säure, ausgewählt aus der Gruppe bestehend aus Chlorwasserstoff-, Bromwasserstoff-, Perchlor-, Schwefel-, Salpeter-, Phosphor-, Essig-, Propion-, Glykol-, Milch-, Bernstein-, Malein-, Fumar-, Äpfel-, Wein-, Zitronen-, Benzoe-, Mandel-, Methansulfon-, Hydroxyethansulfon-, Benzensulfon-, Oxal-, Pamoa-, 2-Naphthalensulfon-, p-Toulensulfon-, Cyclohexansulfamin-, Salizyl-, Saccharin- oder Trifluoressigsäure, gebildet wird.

3. Die Verwendung nach Anspruch 1 oder 2, wobei das Dipeptiyl Peptidase IV-ähnliche Enzym ausgewählt ist aus der Gruppe bestehend aus Fibroblast Aktivierungsprotein α, Dipeptidyl Peptidase IV β, Dipeptidyl Aminopeptidase-ähnlichem Protein, N-acetylierte alpha-verknüpfte saure Peptide spaltende Dipeptidase, Zellruhestands Prolin-Dipeptidase, Dipeptidyl Peptidase II, Attractin, Dipeptidyl Peptidase IV verwandtem Enzym (DPP8), Dipeptidyl Peptidase 9 (DPP9), KIAA1492, DPRP1, DPRP2 und DPRP3.

4. Die Verwendung nach einem der vorstehenden Ansprüche, wobei die pharmazeutische Zusammensetzung einen pharmazeutisch akzeptablen Träger oder ein pharmazeutisch akzeptables Verdünnungsmittel und eine therapeutisch wirksame Menge des Inhibitors oder ein pharmazeutisch akzeptables Säurezusatzsalz und/oder Solvat davon umfasst.

5. Die Verwendung nach einem der vorstehenden Ansprüche, wobei der Inhibitor in Kombination mit einem pharmazeutisch akzeptablen Träger und/oder Verdünnungsmittel verwendet wird.

## Revendications

1. Utilisation d'au moins un inhibiteur de l'activité de la dipeptidyl peptidase IV (DPIV) ou d'une enzyme de type DPIV pour la préparation d'une composition pharmaceutique pour le traitement de sclérose multiple, dans laquelle l'inhibiteur est un analogue de dipeptide choisi dans le groupe constitué de L-thréo-isoleucyl pyrrolidine, L-allo-isoleucyl thiazolidine, L-allo-isoleucyl pyrrolidine, L-thréo-isoleucyl thiazolidine, L-glutaminyl thiazolidine, L-glutaminyl pyrrolidine, acide L-glutamique thiazolidine, acide L-glutamique pyrrolidine, H-Asn-pyrrolidine, H-Asn-thiazolidine, H-Asp-pyrrolidine, H-Asp-thiazolidine, H-Asp(NHOH)-pyrrolidine, H-Asp(NHOH)-thiazolidine, H-Glu (NHOH) -pyrrolidine, H-Glu (NHOH) -thiazolidine, H-His-pyrrolidine, H-His-thiazolidine, H-Pro-pyrrolidine, H-Pro-thiazolidine, H-Ile-azétidine, H-Val-pyrrolidine, H-Val-thiazolidine, et leurs sels et/ou solvates.

2. Utilisation selon la revendication 1, dans laquelle le sel est formé à partir d'un acide inorganique ou organique choisi dans le groupe constitué d'acide chlorhydrique, bromhydrique, perchlorique, sulfurique, nitrique, phosphorique, acétique, propionique, glycolique, lactique, succinique, maléique, fumarique, malique, tartrique, citrique, benzoïque, mandélique, méthanesulfonique, hydroxyéthanesulfonique, benzènesulfonique, oxalique, pamoïque, 2-naphthalènesulfonique, p-toluènesulfonique, cyclohexanesulfamique, salicylique, saccharinique ou trifluoracétique.

3. Utilisation selon les revendications 1 ou 2, dans laquelle l'enzyme de type dipeptidyl peptidase IV est choisie dans le groupe constitué de protéine d'activation de fibroblaste α, dipeptidyl peptidase IV β, protéine de type dipeptidyl aminopeptidase, dipeptidase acide N-acétylée à liaison α, proline dipeptidase de cellule inactive, dipeptidyl peptidase II, attractine, protéine se rapportant à la dipeptidyl peptidase IV (DPP 8), dipeptidyl peptidase 9 (DPP 9), KIAA1492, DPRP1, DPRP2 et DPRP3.

4. Utilisation selon l'une quelconque des revendications précédentes, dans laquelle ladite composition pharmaceutique comprend un vecteur ou diluant pharmaceutiquement acceptable et une quantité thérapeutiquement efficace dudit inhibiteur ou d'un sel d'addition acide pharmaceutiquement acceptable et/ou solvate de celui-ci.

5. Utilisation selon l'une quelconque des revendications précédentes, dans laquelle l'inhibiteur est utilisé en combinaison avec un vecteur et/ou diluant pharmaceutiquement acceptable.
